# EUROPEAN PATENT SPECIFICATION

(11) **EP 1 829 581 B1**
(45) Date of publication and mention of the grant of the patent: **17.06.2009**
(21) Application number: 06425143.2
(22) Date of filing: 02.03.2006
(51) Int. Cl.: A61N 5/06, B05B 1/26, B05B 1/18, F21V 33/00

(54) **Shower head having a unit for chromotherapy**
Brause mit Einrichtung zur Farblichttherapie
Pomme de douche avec un disposif de chromothérapie

(43) Date of publication of application: 05.09.2007
(73) Proprietor: Jacuzzi Europe Spa, 33098 Valvasone (Pordenone) (IT)
(72) Inventor: Carrara, Adriano, 33030 S. Odorico di Flaibano (Udine) (IT); Furlan, Livio, 33170 Pordenone (IT)
(74) Representative: Agostini, Agostino

(56) References cited:
- DE-A1- 10 312 866
- DE-U1- 20 101 460
- FR-A- 2 418 677
- GB-A- 797 273
- US-A- 5 685 489
- US-A- 2005 263 619

## Description

### Field of the invention

The disclose invention concerns a unit that combines a chromotherapy device and a shower diffuser.

### State of the art

It is well known that in the light spectrum, any colour, in particular any iris colour, corresponds to a different wavelength. Chromotherapy consists in exploiting light of one or more specific colours, produced by an electric light source, to irradiate a person's entire body or occasionally only part of the same for a given length of time. Depending on their wave length, the electromagnetic waves penetrate more or less deeply into the skin tissues, thus physically and even more psychically benefiting the person.

The addition to sanitary fixtures (rubs and shower cabinets) of chromotherapy devices, for instance by integrating them in the functional elements these fixtures are commonly equipped with, is also known.

The patent US-A-6 021 960 discloses a device consisting in a shower water diffuser wherein a light source (an incandescent lamp or a LED), with its power supply, is housed in a watertight and opaque enclosure behind the perforated shower dish. Said enclosure is closed on its front side by a transparent lid incorporating, in case of a non-coloured light source, a coloured filter. The limitations of this device consist in the fact that the light rays parallel to the water sprays are only of a single colour type, and if irradiated from a light source set at a distance from the perforated dish, are unable to provide a full colouring of all the sprays.

The patent application EP-A-1 440 649 discloses a device comprising a shower water diffuser having a relatively compact spraying surface and a lighting unit arranged at a central point above the diffuser (when the device is installed on top of a shower cabinet) or behind the diffuser (when it is installed on a lateral wall), The light group comprises a multiple number of coloured or otherwise colourable LEDs by using an electronic control module. The light irradiated by the LEDs is deviated by a reflective parabola in a direction parallel to the water sprays and forced to pass a plate of transparent or translucent material having a much broader surface than the diffuser. This device can provide the person with a chromotherapy, but cannot illuminate the water, as the luminous rays remain outside the sprays.

The patent application WO-A-2004/082 846, which discloses the preamble of claim 1, is in turn a development of the mentioned EP, from which it differs by the fact that the plate of a transparent or translucent material has the same surface of the underlying (or laying before) perforated diffuser, at a very short yet adequate distance from the latter to create a chamber. Inside the chamber a rubber membrane is seated on the diffuser and fitted with a multiple number of nozzles fitted into the holes of the diffuser. The water fills this chamber before exiting as sprays ejected by the nozzles. The luminous rays, while still deviated by the reflecting parabola in a direction parallel to the water sprays, in this case illuminate the separate water sprays or the entire diffuser, if the diffuser is also made of a transparent or translucent material. The chromotherapeutic effect of this device could nevertheless be poor, because of the light deviation and of the three solid layers (transparent or translucent plate, nozzle membrane and perforated diffuser) that the luminous rays must pass before impinging upon the person's body.

Shower diffusers capable of colouring the water before it exits the spray nozzles are also known, for instance the utility model DE-U-20 101 460 wherein an electronic card for a plurality of LEDs, of even different colours, is mounted behind the transparent material plate fitted with the spray holes. The card's power supply has a miniaturized turbo-generator driven by the flow of water entering the diffuser, which obviously cannot wet the electronic card. The main limitation of this device is that it cannot provide a chromotherapeutic effect without simultaneously operating the shower, while it would be desirable for the user even to use the shower without chromotherapy, and vice versa.

### Scope of the invention

The object of the invention is to offer a simple construction unit combining a chromotherapy device with a shower diffuser in an appropriate unit, without the limitations and other drawbacks mentioned in the patents of the known art. The two functions, chromotherapy and shower, may be used both individually and simultaneously, thus creating a particular "coloured rain" showering effect. The chromotherapy device may further be used inside a multifunctional box together with a steam bath, thus associating the beneficial effects of a Turkish bath with the relaxing and invigorating effects of chromotherapy.

These and other objects are achieved by a unit with the characteristics of the appended claims.

### Brief description of the figures

The following description of a preferred embodiment is offered only for exemplifying but non-limiting purposes of the invention, and refers to the enclosed drawings, where:
- Figure 1 is an exploded view of the unit according to the invention;
- Figure 2 is a partially sectionalized front view of the unit of Figure 1;
- Figure 3 is a sectionalized view along the line III - III of Figure 2,
which allow appreciating the constructive and functional characteristics as well as the advantages of the present invention.

### Description of a form of embodiment

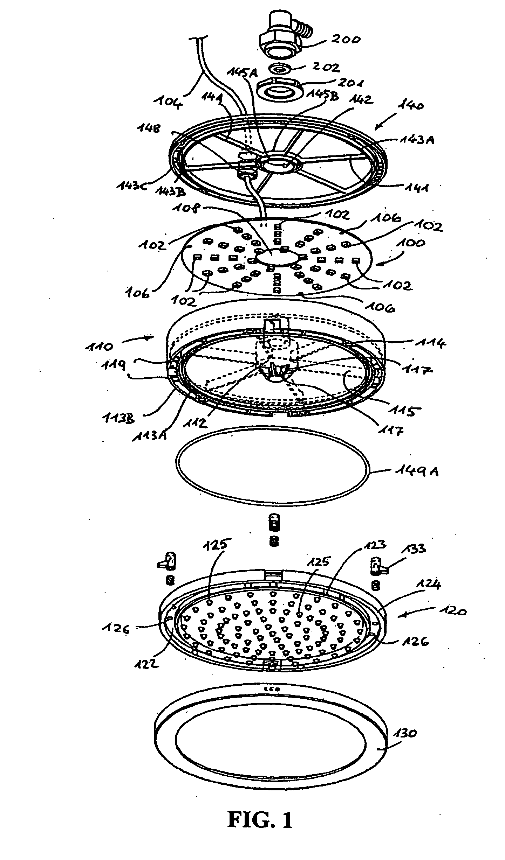

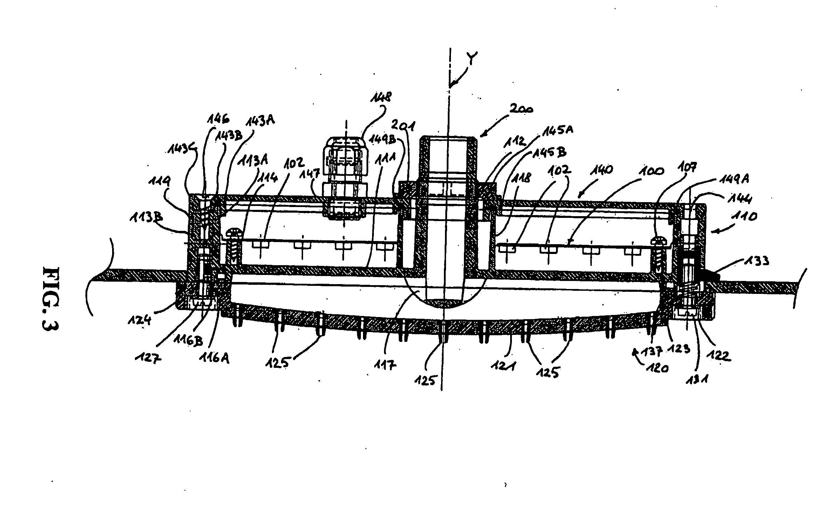

As shown in Figure 1 and 3, a chromotherapy device according to the invention consists essentially of an electronic card 100 mounting a plurality of LEDs 102, which are connected to each other by printed circuits connected to a suitable and in itself well known external power supply (which is in turn fed by the electrical supply network) by a cable 104. In the electronic card 100, having a round disc shape with a few perforations 106 for fastening screws 107 along its rim - as shown in Figure 3 - the LEDs 102 are arranged in radial rows starting from a circular central opening 108. For simplicity, the printed circuits and power supply are not shown, as they are of an entirely conventional type and preferably comprise a control device designed to vary the lighting intensity and/or the colour of the various LEDs.

According to the invention, the mentioned chromotherapy device is integrated in a single unit with the shower water diffuser, being housed within the same enclosure.

The enclosure consists essentially of a few elements symmetrical to an axis Y which, according to the mode of installation of the unit, is either vertical (as shown in the enclosed drawing) or horizontal. The mentioned symmetrical elements are an upper shell 110, a lower shell 120, a lower covering ring 130 and a cover 140.

As shown in greater detail in Figure 1, the upper shell 110, being preferably made as a single injection-moulded piece, consists of a round base 111, a centrally arranged sleeve 112 and a peripheral wall consisting of a pair of cylindrical crowns 113A-B, the said sleeve and crowns extending from both the distal and proximal face of the base.

The base 111, which is made of a transparent or translucent material, forms the partitioning wall between the chromotherapy device and the underlying shower water diffuser described below.

Two fastening turrets 114 and a multiple number of radial stiffening ribs 115 (extending inward from the innermost peripheral crown 113A, as shown in Figure 1) are integrally made at the distal face of the base 111. A second pair of cylindrical crowns 116A-B are integrally made at the opposite or proximal face of the base 111. The axis of both pairs of cylindrical crowns 113A-B and 116A-B is the above mentioned axis Y and, as shown in Figure 3, their axial extension starting from the corresponding bottom face 111, namely their height, is such that the first pair of cylindrical crowns 113A-B exceed in height the turrets 114, which in turn exceed in height the second pair of cylindrical crowns 116A-B.

The electronic card 100 is housed inside the upper shell 110 so as to lean against the radial ribs 115 and be fixed in position by the mentioned fastening screws 107, whose stems cross the holes 106 and engage with the peripheral turrets 114. As can be appreciated from Figure 3, the electronic card 100 is parallel and very close to the base 111.

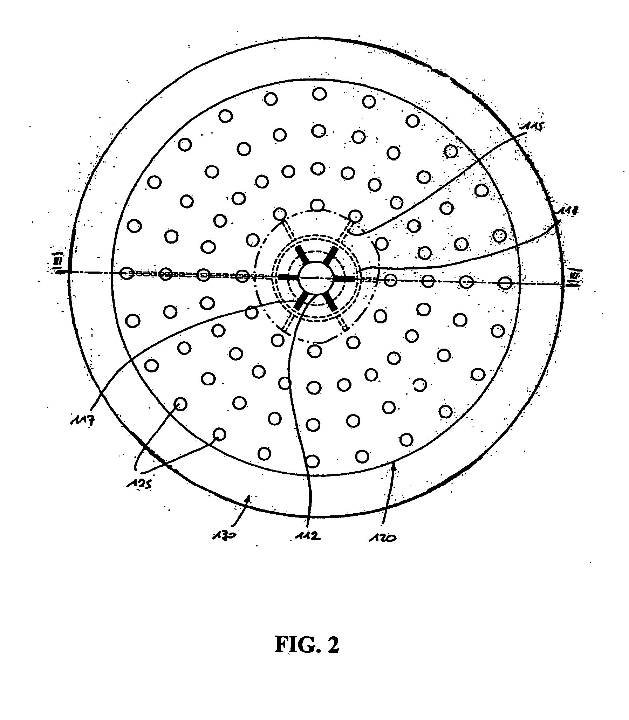

A first part of the sleeve 112 projecting from the distal face of the base 111 serves to connect the unit to a fitting 200 which forms a portion of the water feeding system, through an annular gasket 202 - see Figure 1 -, while a second portion of the same sleeve 112, projecting from the proximal face of the base 111, ends in a number of radial water outlets separated by deflectors 117 of a triangular shape - see Figure 1 and 2. The sleeve 112 thus fulfils the double function as an internal collector and distributor of the water before this falls onto the user's body, as better outlined below,

Around the sleeve 112, the base 111 is integrally provided at its distal face with a second sleeve 118, wherein the mentioned stiffening ribs 115 end. Together with the first pair of cylindrical crows 113A-B, said second sleeve 118 supports the cover 140, which ensures the required protection for the electronic card 100. The mentioned cover has for this purpose a central opening 142 for the first sleeve 112, a triple peripheral cylindrical crown 143A-B-C, a double internal cylindrical crown 145A-B and a second opening 147, which is offset and has an inwardly turned rim to insert a traditional clamp 148 for the supply cable 104. The radial spacing between the two innermost cylindrical crowns 143A-B is the same as for the peripheral cylindrical crowns 113A-B of the upper shell 110, so as leave room for both a row of holes 146 of screws which engage in bushings 119 provided between said crowns 113A-B of the upper shell 110, as well as for an O-ring 149A held in place by a third cylindrical crown 143C of the cover 140. The radial spacing between the two inner cylindrical crowns 145A-B of the cover 140 allows seating a second O-ring 149B along the free upper rim of the second sleeve 118 - see Figure 1 and 3.

The injection-molded lower shell 120 comprises a circular and slightly concave ring 121 made of a translucent material and a frame composed of a flat flange 122 interposed between a pair of cylindrical crowns 123 and 124. The disc 121 is a true shower diffuser, as it sustains a multiple number of nozzles 125 distributed over two radial rows, which constitute the openings through which the water falls along a direction parallel to the axis Y. The nozzles 125 are fabricated of thermoplastic rubber, preferably overinjected onto the disc 121, or alternatively, the disc 121 is fitted with a multiple number of through holes in which an equal number of separately moulded nozzles are fitted.

As can be appreciated above all from Figure 3, a chamber 135 remains between the distal face of the disc 121 of the lower shell 120 and the proximal face of the base 111 of the upper shell 110, the hydraulic tightness of the chamber 135 being ensured by an O-ring 137 seated inside the third pair of the upper shell's 110 cylindrical crowns 116A-B. The size and shape of the chamber 135 play an important role and must therefore be carefully designed, while taking into account that the lower portion of the sleeve 112 with its radial deflectors 117 is set at the centre of the chamber. According to this invention, it is precisely the volume of water held in the chamber 135, before falling through the nozzles 125, that ensures the optimum diffusion of the LED-emitted light for the exploitation of the chromotherapy when it occurs simultaneously with the shower.

The lower shell 120 is joined to the upper shell 110 by a multiple number of screws 127, whose stems cross the holes 126 at the flange 122 and engage in the bushings 119 provided between the peripheral cylindrical crowns 113A-B. The heads of the screws 127 remain inside the space between the peripheral cylindrical crowns 123 and 124 of the lower shell 120, and are protected from water by a covering ring 130, which is snap-mounted on the outermost crown 124 - see Figure 1 and 3.

In order to be fastened in a removable manner to the installation zone (which is mostly a sanitary fixture, for instance the roof or side wall of a shower cabinet) the use of a number of wing nuts 133 is foreseen. These are adjustable by associated spring-loaded screws 131 well known as such, whose stems are seated in holes arranged between the peripheral cylindrical crowns 113A-B of the upper shell 110.

Whenever the chromotherapy occurs simultaneously with the shower, the combined unit of the present invention operates as follows.

Based on appropriate commands from outside the unit (water mixer and electrical controls) the water is conveyed through the fitting 200 and the sleeve 112 and the LEDs 102 are switched on. After an appropriate deflection by the deflectors 117, the water fills the chamber 135 and assumes the colour of the LEDs, not merely inside the chamber 135 but also, after filling the chamber, when it falls through the nozzles 125 onto the user's body. Thanks to the fact that the electronic plate 100 is parallel to the base 111 and has substantially the same extension of the latter, and that the LEDs are arranged on the plate 110 in radial rows similar to those of the nozzles 125 on the water diffuser 121, the light used for the chromotherapy is in fact not appreciably deviated with respect to the LEDs' direction of emission, which is aligned with the Y-axis, with a beneficial "rain effect".

This generates a synergic effect between the chromotherapy and shower action, to the full benefit of the user.

It is nevertheless clear that the operation of the chromotherapy device (namely the LEDs energization) is entirely independent from the operation of the shower water diffuser or of any other water and steam dispensing unit in the sanitary fixture, whereof the invention's combined unit is a part.

While the foregoing description refers to a preferred embodiment, it is understood that the invention may be implemented in different forms and variants within the scope of the appended claims.

## Claims

1. Combined unit comprising in a single enclosure:
- a chromotherapy device suitable for connection to a power supply outside the unit and comprising
- a plurality of LEDs (102) **arranged in radial rows onto a plane of an electronic card (100),**
- a shower water diffuser (121) **from which the water falling onto the user's body occurs along an axis (Y);**
- a watertight partitioning wall (111), which is fabricated from a transparent or translucent material, between the chromotherapy device and the water diffuser (121),
- a chamber (135) comprised between the water diffuser (121) and the partitioning wall (111),
- water inlet means (112) **in the form of a sleeve whose lower portion opens in the center of said chamber;**
**characterized in that** the plane (100) on which the LEDs (102) are arranged is
- coextensive with the water diffuser (121),
- substantially parallel to the water diffuser (121) and to the partitioning wall (111), in such a way that the direction of emission of the light by the LEDs (102) **is aligned to said axis (Y),**
and further **characterized in that**
the lower portion of said sleeve (112) projects in the center of said chamber and said sleeve ends with radial deflectors (117) arranged in said chamber (135) with the result that during the use the water fills said chamber (135) and the water in the chamber and falling from the diffuser (121) onto the user's body is coloured by the LEDs when they are energized.

2. Combined unit according to claim 1, **characterized in that** the radial deflectors (117) are of a triangular shape and the water diffuser consists of a plate (121) with a plurality of nozzles (125) overinjected onto said plate or fitted into holes provided in the same plate whereby the water falls onto the user's body through said nozzles (125).

3. Combined unit according to claim 2, **characterized in that** said first and second shell (110, 120) are joined by removable connecting means (127) distributed over respective peripheral zones (113A-B, 123, 124).

4. Combined unit according to claim 3, **characterized in that** the said partitioning wall (111) between the chromotherapy device and the water diffuser (121) is the base of said first shell (110).

5. Combined unit according to claim 4, **characterized in that** said first shell (110) also comprises, at a central point, a first sleeve (112) which is the said water inlet means, with a first portion projecting from the distal face of said base (111) and a second portion projecting from the proximal face of the same base.

6. Combined unit according to claim 5, **characterized in that** the first portion of said first sleeve (112) is suitable for connecting to the water supply system, while said second portion of the same sleeve (112) comprises the water deflectors (117) which are arranged in said chamber (135).

7. Combined unit according to claim 6, **characterized in that** said first shell (110) also comprises, at a central point, a second sleeve (118) arranged coaxially and externally to the first sleeve (112), wherefrom a plurality of stiffening ribs (115) extend between the peripheral zone (113A-B) of the same first shell (110) and said second sleeve (118).

8. Combined unit according to claim 1, **characterized in that** it is fitted with adjustable fastening means (133) for permitting its fixation onto a mounting zone, for instance the roof or side wall of a shower cabinet.

9. Combined unit according to claim 1, **characterized in that** said plane has a round disc shape with a circular central opening into which said sleeve is inserted.

10. Combined unit according to claim 1, **characterized in that** said sleeve (112) ends in a number of water outlets separated by said deflectors.

## Patentansprüche

1. Kombinationseinheit, die in einem einzelnen Gehäuse enthält:
eine Chromotherapievorrichtung, die zum Anschließen an eine Leistungsversorgung außerhalb der Einheit geeignet ist und enthält:
eine Mehrzahl von LEDs (102), die in radialen Reihen auf einer Fläche einer Elektronikkarte (100) angeordnet sind,
einen Duschwasserdiffusor (121), von dem aus das Wasser, das auf den Körper des Benutzers fällt, entlang einer Achse (Y) austritt,
eine wasserdichte Trennwand (111), die aus einem transparenten oder lichtdurchlässigen Material hergestellt ist, zwischen der Chromotherapievorrichtung und dem Wasserdiffusor (121),
eine Kammer (135), die zwischen dem Wasserdiffusor (121) und der Trennwand (111) enthalten ist,
ein Wassereinlassmittel (112) in Form einer Hülse, deren unterer Abschnitt sich in der Mitte der Kammer öffnet,
**dadurch gekennzeichnet, dass** die Fläche (100), auf der die LEDs (102) angeordnet sind,
koextensiv mit dem Wasserdiffusor (121) ist und
im Wesentlichen parallel zu dem Wasserdiffusor (121) und zu der Trennwand (111) ist, so dass die Richtung der Abstrahlung des Lichts durch die LEDs (102) mit der Achse (Y) ausgerichtet ist, und
weiter **dadurch gekennzeichnet, dass** der untere Abschnitt der Hülse (112) in der Mitte der Kammer vorspringt und dass die Hülse in Radialablenkern (117) endet, die in der Kammer (135) angeordnet sind, so dass das Wasser während der Verwendung die Kammer (135) und füllt und das Wasser in der Kammer, das von dem Diffusor (121) auf den Körper des Benutzers fällt, durch die LEDs gefärbt ist, wenn sie mit Energie versorgt sind.

2. Kombinationseinheit gemäß Anspruch 1, **dadurch gekennzeichnet, dass** die Radialablenker (117) eine Dreiecksform haben und der Wasserdiffusor aus einer Platte (121) mit einer Mehrzahl von Düsen (125) besteht, die in die Platte überinjiziert sind oder in Löcher eingepasst sind, die in derselben Platte bereitgestellt sind, wobei das Wasser durch diese Düsen (125) auf den Körper des Benutzers fällt.

3. Kombinationseinheit gemäß Anspruch 2, **dadurch gekennzeichnet, dass** die erste und zweite Hülle (110, 120) durch entfernbare Verbindungsmittel (127) verbunden sind, die über jeweilige Randzonen (113A-B, 123, 124) verteilt sind.

4. Kombinationseinheit gemäß Anspruch 3, **dadurch gekennzeichnet, dass** die Trennwand (111) zwischen der Chromotherapievorrichtung und dem Wasserdiffusor (121) die Basis der ersten Hülle (110) ist.

5. Kombinationseinheit gemäß Anspruch 4, **dadurch gekennzeichnet, dass** die erste Hülle (110) in einem zentralen Punkt auch eine erste Hülse (112) enthält, die das Wassereinlassmittel ist, mit einem ersten Abschnitt, der von der distalen Fläche der Basis (111) aus vorspringt, und einem zweiten Abschnitt, der von der proximalen Fläche derselben Basis aus vorspringt,

6. Kombinationseinheit gemäß Anspruch 5, **dadurch gekennzeichnet, dass** der erste Abschnitt der ersten Hülse (112) geeignet ist, ein Wasserzuführsystem anzuschließen, während der zweite Abschnitt derselben Hülse (112) die Wasserablenker (117) aufweist, die in der Kammer (135) angeordnet sind.

7. Kombinationseinheit gemäß Anspruch 6, **dadurch gekennzeichnet, dass** die erste Hülle (110) in einem zentralen Punkt auch eine zweite Hülse (118) enthält, die koaxial zu und außerhalb der ersten Hülle angeordnet ist und von der aus sich eine Mehrzahl von Versteifungsrippen (115) zwischen der der Randzone (113A-B) derselben ersten Hülle (110) und der zweiten Hülse (118) erstrecken.

8. Kombinationseinheit gemäß Anspruch 1, **dadurch gekennzeichnet, dass** sie mit einem einstellbaren Befestigungsmittel (133) versehen ist, um ihre Befestigung in einem Montagebereich, beispielsweise der Decke oder der Seitenwand einer Duschkabine, zu ermöglichen.

9. Kombinationseinheit gemäß Anspruch 1, **dadurch gekennzeichnet, dass** die Fläche die Form einer runden Scheibe mit einer kreisförmigen Mittelöffnung hat, in die die Hülse eingeführt wird.

10. Kombinationseinheit gemäß Anspruch 1, **dadurch gekennzeichnet, dass** die Hülse (112) in einer Anzahl von Wasserauslässen endet, die durch die Ablenker getrennt sind.

## Revendications

1. Unité combinée comprenant dans une enceinte unique :
- un dispositif de chromothérapie approprié pour être raccordé à une alimentation à l'extérieur de l'unité et comprenant
- une pluralité de LEDs (102) disposées en rangées radiales sur un plan d'une carte électronique (100),
- un diffuseur d'eau de douche (121) à partir duquel l'eau tombe sur le corps de l'utilisateur le long d'un axe (Y) ;
- une cloison étanche à l'eau (111), qui est fabriquée à partir d'un matériau transparent ou translucide, entre le dispositif de chromothérapie et le diffuseur d'eau (121),
- une chambre (135) comprise entre le diffuseur d'eau (121) et la cloison (111),
- un moyen d'admission d'eau (112) sous la forme d'un manchon dont la partie inférieure s'ouvre au centre de ladite chambre ;
**caractérisée en ce que** le plan (100) sur lequel les LEDs (102) sont disposées
- coïncide avec le diffuseur d'eau (121),
- est sensiblement parallèle au diffuseur d'eau (121) et à la cloison (111), de telle sorte que la direction d'émission de la lumière par les LEDs (102) est alignée avec ledit axe (Y),
et **caractérisée en outre en ce que** la partie inférieure dudit manchon (112) dépasse au centre de ladite chambre et ledit manchon se termine par des déflecteurs radiaux (117) agencés dans ladite chambre (135), ce qui entraîne pendant l'utilisation le remplissage de ladite chambre (135) par de l'eau et la coloration de l'eau dans la chambre s'écoulant du diffuseur (121) sur le corps de l'utilisateur par des LEDs lorsqu'elles sont alimentées.

2. Unité combinée selon la revendication 1, **caractérisée en ce que** les déflecteurs radiaux (117) sont de forme triangulaire et le diffuseur d'eau est constitué d'une plaque (121) avec une pluralité de buses (125) surinjectées sur ladite plaque ou ajustées dans des trous prévus dans la même plaque de telle sorte que l'eau s'écoule sur le corps de l'utilisateur par lesdites buses (125).

3. Unité combinée selon la revendication 2, **caractérisée en ce que** ladite première et ladite seconde enveloppe (110, 120) sont jointes par des moyens de raccord amovibles (127) distribués sur des zones périphériques (113A à B, 123, 124) respectives.

4. Unité combinée selon la revendication 3, **caractérisée en ce que** ladite cloison (111) entre le dispositif de chromothérapie et le diffuseur d'eau (121) constitue la base de ladite première enveloppe (110).

5. Unité combinée selon la revendication 4, **caractérisée en ce que** ladite première enveloppe (110) comprend également, en un point central, un premier manchon (112) qui constitue ledit moyen d'admission d'eau, une première partie dépassant de la face distale de ladite base (111) et une seconde partie dépassant de la face proximale de la même base.

6. Unité combinée selon la revendication 5, **caractérisée en ce que** la première partie dudit premier manchon (112) est appropriée pour être raccordée à un système d'alimentation en eau, alors que ladite seconde partie du même manchon (112) comprend les déflecteurs d'eau (117) qui sont agencés dans ladite chambre (135).

7. Unité combinée selon la revendication 6, **caractérisée en ce que** ladite première enveloppe (110) comprend également, en un point central, un second manchon (118) agencé de manière coaxiale et externe au premier manchon (112), à partir duquel s'étendent une pluralité de nervures de raidissement (115) entre la zone périphérique (113A à B) de la même première enveloppe (110) et ledit second manchon (118).

8. Unité combinée selon la revendication 1, **caractérisée en ce qu'**elle s'ajuste avec des moyens de fixation ajustables (133) pour permettre sa fixation sur une zone de montage, par exemple le plafond ou le mur latéral d'une cabine de douche.

9. Unité combinée selon la revendication 1, **caractérisée en ce que** ledit plan a la forme d'un disque avec une ouverture centrale circulaire dans laquelle est inséré ledit manchon.

10. Unité combinée selon la revendication 1, **caractérisée en ce que** ledit manchon (112) se termine en un certain nombre d'évacuations d'eau séparées par lesdits déflecteurs.
